# FASCICULE DE BREVET EUROPEEN

(11) **EP 0 857 971 B1**
(45) Date de publication et mention de la délivrance du brevet: **18.12.2002**
(21) Numéro de dépôt: 98400266.7
(22) Date de dépôt: 06.02.1998
(51) Int. Cl.: G01N 33/50

(54) **Procédé d'évaluation du potentiel sensibilisant et/ou irritant et/ou allergène d'un produit**
Verfahren zur Bewertung des sensibilisierenden und/oder irrtierenden und/oder allergenen Potenzials eines Produkts
Process to evaluate the sensitizing and/or irritation and/or allergenic potential of a product

(30) Priorité: 11.02.1997 FR 9701576
(43) Date de publication de la demande: 12.08.1998
(73) Titulaire: L'OREAL, 75008 Paris (FR)
(72) Inventeur: Schmidt, Rainer, 92140 Clamart (FR); Regnier, Marcelle, 75018 Paris (FR)
(74) Mandataire: Tezier Herman, Béatrice

(56) Documents cités:
- EP-A- 0 497 399
- WO-A-90/02796
- A. RAMBUKKANA ET AL.: "Effects of contact allergens on human Langerhans cells in skin organ culture: migration, modulation of cell surface molecules and early expression of interleukin-1beta protein" LABORATORY INVESTIGATION, vol. 74, no. 2, 1996, WASHINGTON DC USA, pages 422-436, XP002043544
- F.H.M. PISTOOR ET AL.: "Novel predictive assay for contact allergens using human skin explant cultures." AMERICAN JOURNAL OF PATHOLOGY, vol. 149, no. 1, 1 juillet 1996, WASHINGTON DC USA, pages 337-343, XP002043821

## Description

L'invention a pour objet un procédé d'évaluation du potentiel sensibilisant et/ou irritant et/ou allergène d'un produit.

La peau constitue l'organe le plus important de l'organisme et est reconnue comme l'un des principaux éléments actifs du système de défense immunitaire.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.
Les cellules de Langerhans sont impliquées dans les défenses immunitaires de la peau. On sait depuis longtemps que celles-ci occupent une place essentielle dans les défenses immunitaires de l'hôte, en particulier comme première barrière face aux agressions extérieures.

Les cellules de Langerhans sont des cellules dérivées de la moelle osseuse qui peuvent être caractérisées par l'expression du marqueur antigénique CD1a (cellules CD1a positives). Dans l'épiderme, les cellules de Langerhans épidermique se caractérisent également par la présence de granules de Birbeck (Rowden et coll., Nature 268 : 247-248, 1977).
Elles jouent un rôle décisif dans l'initiation des réponses immunitaires dirigées contre les antigènes introduits dans la peau ou nouvellement générés par celle-ci. Mises en contact avec un allergène, les cellules de Langerhans migrent vers les ganglions où elles déclenchent les réactions spécifiques des cellules-T. A ce titre elles sont donc assimilées aux cellules de présentation des antigènes, essentielles au bon fonctionnement des lymphocytes T.

Ainsi, la principale fonction des cellules de Langerhans est de fournir un signal sensitif dans la réponse immunitaire de la peau induite contre une grande variété d'antigènes incluant les allergènes de contact, les antigènes tumoraux et les micro-organismes.
On peut donc en conclure que ces cellules interviennent probablement dans un grand nombre de pathologies de la peau.

Par ailleurs, l'industrie en général, et celle des cosmétiques en particulier, introduit de plus en plus souvent des composés nouveaux dans ses compositions. Un des problèmes majeurs auquel elle est confrontée est alors l'évaluation des effets néfastes que ces composés pourraient induire au contact de la peau en particulier en terme de sensibilisation de contact.
Il est donc essentiel de pouvoir évaluer simplement et rapidement le potentiel sensibilisant et/ou irritant et/ou allergène de tels produits.

Pour des raisons d'éthique, il est bien évident que de telles évaluations ne peuvent se faire sans difficulté sur l'homme et qu'il est souhaitable qu'elles ne se fassent pas sur des modèles animaux.

On comprend l'importance et la nécessité de disposer d'un modèle *in vitro* permettant une évaluation sans danger, rapide, fiable et peu coûteuse des produits à risque. Un tel modèle présenterait en outre l'avantage supplémentaire de permettre d'évaluer tout au moins dans des études préliminaires un grand nombre de produits sans difficulté.

Un des principaux obstacles à l'élaboration d'un tel modèle est constitué par le fait que, dans la peau, les cellules de Langerhans sont des cellules ayant subi un programme de différenciation particulier. Rambukkana et collaborateurs (Laboratory Investigation, Vol. 74, 1996, p. 422-436) suggèrent un système de culture de peau utilisant des cellules de Langerhans cultivées simultanément avec des autre cellules de peau comme des kératinocytes. L'utilisation de précurseurs de cellules de Langerhans n'est pas mentionée. Le document WO-A-90 02796 suggère d'ajouter, dans un système tridimensionnel de culture de peau, à des cultures de kératinocytes et de mélanocytes, des cellules de Langerhans isolées à partir de prélèvements de peau fraîche. Ce même document précise que la croissance en culture de telles cellules est difficile. Il s'avère en fait que les cellules de Langerhans purifiées à partir de prélèvement d'épiderme sont des cellules CD1a positives, issues de précurseurs qui ont progressé dans leur cycle de différenciation jusqu'à devenir des cellules qui présentent des granules de Birbeck. Ces cellules une fois isolées ne progressent plus dans leur cycle de différenciation et ne prolifèrent plus.
La culture *in vitro* de telles cellules se résume à un maintien en survie du fait de l'incapacité pour ces cellules de se multiplier. En fait ces cellules s'arrêtent et meurent sans avoir rempli leur rôle. Il en est de même lorsque ces cellules sont introduites dans un modèle de peau reconstruite. Ainsi, l'équivalent de peau obtenu, même s'il contient des cellules de Langerhans, ne pourrait permettre l'évaluation du potentiel sensibilisant et/ou irritant et/ou allergène d'un produit puisqu'il ne contient pas de cellules de Langerhans actives.

La demanderesse a maintenant découvert qu'il est possible d'induire spontanément la différenciation des précurseurs de cellules de Langerhans en les cultivant en présence de kératinocytes. Sur cette base, la demanderesse a élaboré un procédé d'évaluation du potentiel sensibilisant et/ou irritant et/ou allergène d'un produit.

Ainsi, l'invention a pour objet un procédé d'évaluation du potentiel sensibilisant et/ou irritant et/ou allergène d'un produit, caractérisé par le fait que l'on cultive simultanément au moins des kératinocytes et des précurseurs de cellules de Langerhans pendant un temps suffisant pour induire la différenciation des précurseurs de cellules de Langerhans, que l'on met en contact la culture et le produit à tester pendant un temps suffisant, que l'on mesure la variation d'un marqueur du potentiel sensibilisant et/ou irritant et/ou allergène dudit produit et que l'on évalue les résultats de cette mesure par rapport à un témoin.

Les kératinocytes utilisés selon l'invention peuvent être préparés selon toute méthode connue de l'art antérieur. On peut citer à titre d'exemple la culture à partir d'épiderme dissocié provenant de prélèvement de peau normale ou pathologique ou la culture de kératinocytes issus de la gaine de follicules pileux normaux ou pathologiques.

Préférentiellement, selon l'invention les kératinocytes utilisés sont préparés à partir d'épiderme dissocié provenant de prélèvement de peau normale ou pathologique, selon la méthode décrite dans Régnier et collaborateurs, Frontier of Matrix Biology, Vol.9, 4-35 (Karger, Basel 1981).

Avantageusement, les kératinocytes utilisés selon l'invention sont des kératinocytes humains.

Les précurseurs de cellules de Langerhans peuvent être toute cellule souche apte à se différencier sous l'effet d'une induction en cellules de Langerhans, c'est à dire apte à se différencier en cellules CD1a positives. Avantageusement, ces précurseurs peuvent être des cellules hématopoïétiques CD34⁺ (Caux et collaborateurs, Nature, Vol. 360, Nov. 1992, 258).

Les précurseurs de cellules de Langerhans peuvent être purifiés à partir des tissus dans lesquels ils se trouvent naturellement, parmi lesquels on peut citer la moelle osseuse, le sang périphérique, le sang de cordon ombilical.

Préférentiellement, on utilise selon le procédé de l'invention des précurseurs de cellules de Langerhans préparés à partir de sang périphérique ou de sang de cordon ombilical et encore plus préférentiellement des précurseurs préparés à partir de sang de cordon ombilical.

Avantageusement, les précurseurs de cellules de Langerhans utilisés selon l'invention sont d'origine humaine.

Toute méthode de purification peut être utilisée à cette fin. On peut citer, par exemple, celle décrite dans Caux et collaborateurs, Nature, Vol. 360, Nov. 1992, 258.

Selon l'invention, le rapport entre le nombre de kératinocytes et les précurseurs de cellules de Langerhans est compris entre les rapports 95/5 et 25/75 en pourcentage du nombre de cellules total dans la culture et préférentiellement entre 75/25 et 35/65 et encore plus préférentiellement ce rapport est de 50/50.

Le milieu nutritif utilisé pour le procédé selon l'invention peut être tout milieu nutritif connu pour sa capacité à permettre la prolifération et la différenciation des kératinocytes. On peut citer à titre d'exemple le milieu Dulbecco modifié Eagle, ou un milieu défini à teneur en calcium variable, tel le milieu décrit par Boyce S.T. et Ham R.G., (J. Tissue Cult. Meth., 1985, 9, 83-93).
Avantageusement, selon l'invention il est possible d'utiliser un mélange de plusieurs milieux nutritifs comme par exemple le mélange milieu Dulbecco modifié Eagle/milieu HAM F12 ou milieu de Rheinwald et Green, (Cell, 1975, 6, 331-334).

Selon l'invention, on cultive simultanément au moins des kératinocytes et des précurseurs de cellules de Langerhans pendant un temps suffisant pour induire la différenciation des précurseurs de cellules de Langerhans. Comme décrit précédemment, les cellules de Langerhans matures sont des cellules, caractérisées par la présence de granules de Birbeck et l'expression du marqueur antigénique CD1a. Ainsi, le temps de culture suffisant correspond généralement au minimum de temps nécessaire à l'apparition de ces caractéristiques. Ainsi, pour donner un ordre de grandeur on cultive simultanément des kératinocytes et des précurseurs de cellules de Langerhans pendant un temps compris entre 1 et 30 jours, préférentiellement pendant un temps compris entre 2 et 13 jours.

Toute manière de mettre en contact les cellules et le produit à tester est utilisable selon l'invention. Mais, préférentiellement, selon l'invention le produit à tester est mis en solution dans le milieu de culture.

Le produit à tester peut se trouver sous toute forme compatible avec sa mise en contact avec les cellules. Principalement, il peut être sous sa forme moléculaire pure ou encore sous la forme d'une composition.

De manière générale, le temps d'incubation du produit à tester avec la culture cellulaire est conditionné par le temps qu'il faut à ladite culture pour répondre au produit avec lequel elle a été mise en contact, c'est à dire au temps qu'il faut pour voir apparaître une modification du niveau d'expression du marqueur du potentiel sensibilisant et/ou irritant et/ou allergène dudit produit.

Ce temps d'incubation peut aller de quelques secondes à plusieurs jours.
A titre indicatif, le temps d'incubation est généralement compris entre 5 secondes et 72 heures, et de préférence entre 1 et 24 heures.

Par marqueur du potentiel sensibilisant et/ou irritant et/ou allergène dudit produit, on entend selon l'invention, tout élément dont la présence, l'absence, la modification de l'expression ou la modification de la distribution peuvent être mesurées, en réponse à la mise en contact de la culture cellulaire avec ledit produit à tester. On peut citer à titre d'exemple de marqueur, et sans limitation, un acide nucléique, une protéine ou un groupe de protéines liées ou non, un ion, un organite cellulaire, un lipide ou un polyoside.

Préférentiellement, ce marqueur est un élément représentant la réaction des cellules de Langerhans au produit à tester. A cet égard, on trouve dans "The Immune Functions of Epidermal Langerhans Cells" (Heidrun Moll, 1995, Springer-Verlag and R.G. Landes Company Editors) toutes les données nécessaires à une bonne compréhension des phénomènes immunitaires impliquant les cellules de Langerhans.
Ainsi, il est connu qu'en réponse à une mise en contact avec un produit présentant un potentiel sensibilisant et/ou irritant et/ou allergène la densité des molécules du complexe majeur d'histocompatibilité (MHC) de classe II ou l'expression de cytokines comme l'interleukine-1α, l'interleukine-1β, le facteur de stimulation de colonies (Granulocyte/Macrophage- Colony Stimulating Factor ou GM-CSF), le facteur de nécrose tumorale (Tumor Necrosis Factor ou TNF-α), la protéine interferon-induite 10 (interferon-induced protein 10 ou IP10), la protéine inflammatoire macrophagique de type 2 (macrophage inflammatory protein 2 ou MIP2), ou l'interferon-γ (IFN-γ) sont modifiés. Par exemple une courte exposition à un allergène entraîne une augmentation du taux d'acides ribonucléiques messagers (ARNm) de IL-1β, TNF-α, IFN-γ, IP-10, MIP-2, ou IL-α.
Préférentiellement, selon l'invention, le marqueur du potentiel sensibilisant et/ou irritant et/ou allergène du produit à tester est l'interleukine1-β et encore plus préférentiellement le taux d'expression des ARNm de l'interleukine-β.

L'activité de la substance à tester est ainsi représentée par la variation du marqueur que l'on aura choisi de doser.
Par variation, on entend toute modification de la quantité, de la concentration, de la répartition du marqueur que l'on dose.

Pour cela, le procédé selon l'invention comprend une étape de dosage du marqueur du potentiel sensibilisant et/ou irritant et/ou allergène du produit à tester.

Après incubation, ce dosage peut s'effectuer directement sur le milieu de culture pour les éléments excrétés par la culture ou dans les cellules pour les éléments non excrétés.

Ainsi, et plus particulièrement dans le cas où l'élément recherché n'est pas excrété, on peut envisager une étape supplémentaire avant le dosage, au cours de laquelle les cellules sont broyées, afin de rendre plus accessible le marqueur de l'activité de la substance testée à doser.

Bien évidemment, quel que soit le mode de mise en oeuvre du procédé selon l'invention, toute méthode de dosage connue par l'homme du métier peut être utilisée.
On peut, à titre d'exemple et sans limitation, citer les méthodes de dosage des protéines ou des acides nucléiques par colorimétrie, par électrophorèse, par transcription reverse et amplification par la technique des polymérisations en chaîne, la spectrographie de masse, la chromatographie (en phase gazeuse ou sur plaque), méthodes immunologiques, ou encore la microscopie optique ou électronique pour la mesure de la quantité d'un organite.

Le résultat du dosage, qui représente la variation du marqueur du potentiel sensibilisant et/ou irritant et/ou allergène du produit à tester que l'on a choisi de doser, n'est en soi pas exploitable directement. Il ne devient intéressant que dans la mesure où il est comparé au résultat du même dosage effectué dans les mêmes conditions, mais en l'absence de toute mise en contact de la culture avec le produit à tester.
C'est ainsi que le procédé selon l'invention inclut une étape au cours de laquelle on évalue les résultats du dosage par rapport à un témoin.
L'homme du métier détermine aisément, par habitude, la nature du témoin nécessaire dans la mise en oeuvre du procédé. Préférentiellement, le témoin est une culture identique en l'absence de mise en contact avec le produit à tester.

Un avantage de l'invention est qu'elle procure, dans le domaine de l'immunologie et ou l'allergologie, une méthode de test d'un produit éventuellement sensibilisant et/ou irritant et/ou allergène à la fois simple, rapide et efficace.

Il est aisément compréhensible que le procédé de l'invention peut également permettre l'évaluation de produits capables de moduler, voire inhiber, l'effet sensibilisant et/ou irritant et/ou allergène d'un autre produit.
Dans cette application particulière, le procédé de l'invention précédente comprend une étape supplémentaire qui correspond à l'application d'un produit capable de moduler, voire inhiber, l'effet sensibilisant et/ou irritant et/ou allergène avant, pendant ou après la mise en contact du produit sensibilisant et/ou irritant et/ou allergène
Ainsi, le témoin est alors une culture en présence du produit sensibilisant et/ou irritant et/ou allergène de référence, mais en l'absence du produit capable de moduler voire inhiber l'effet sensibilisant et/ou irritant et/ou allergène à évaluer.

## Revendications

1. Procédé d'évaluation du potentiel sensibilisant et/ou irritant et/ou allergène d'un produit, **caractérisé par le fait que** l'on cultive simultanément au moins des kératinocytes et des précurseurs de cellules de Langerhans pendant un temps suffisant pour induire la différenciation des précurseurs de cellules de Langerhans, que l'on met en contact la culture et le produit à tester pendant un temps suffisant, que l'on mesure la variation d'un marqueur du potentiel sensibilisant et/ou irritant et/ou allergène dudit produit et que l'on évalue les résultats de cette mesure par rapport à un témoin.

2. Procédé selon la revendication précédente, **caractérisé par le fait que** le rapport entre le nombre de kératinocytes et les précurseurs de cellules de Langerhans est compris entre les rapports 95/5 et 25/75 en pourcentage du nombre total de cellules dans la co-culture et préférentiellement entre 75/25 et 35/65 et encore plus préférentiellement ce rapport est de 50/50.

3. Procédé selon l'une quelconque des revendications précédentes, **caractérisé par le fait que** les kératinocytes sont obtenus par la culture à partir d'épiderme dissocié provenant de prélèvement de peau normale ou pathologique ou par la culture de kératinocytes issus de la gaine de follicule pileux normaux ou pathologiques.

4. Procédé selon la revendication précédente, **caractérisé par le fait que** les kératinocytes sont obtenus à partir de prélèvement de peau normale ou pathologique.

5. Procédé selon l'une quelconque des revendications précédentes, **caractérisé par le fait que** les kératinocytes sont des kératinocytes humains.

6. Procédé selon l'une quelconque des revendications précédentes, **caractérisé par le fait que** les précurseurs des cellules de Langerhans sont purifiés à partir de moelle osseuse, de sang périphérique ou de sang de cordon ombilical.

7. Procédé selon la revendication précédente, **caractérisé par le fait que** les précurseurs des cellules de Langerhans sont purifiés à partir de sang de cordon ombilical.

8. Procédé selon l'une quelconque des revendications précédentes, **caractérisé par le fait que** les précurseurs de cellules de Langerhans sont des cellules hématopoïétiques CD34⁺.

9. Procédé selon l'une quelconque des revendications précédentes, **caractérisé par le fait que** les précurseurs des cellules de Langerhans sont d'origine humaine.

10. Procédé selon l'une quelconque des revendications précédentes, **caractérisé par le fait que** le marqueur du potentiel sensibilisant et/ou irritant et/ou allergène est choisi parmi un acide nucléique, une protéine ou un groupe de protéines liées ou non, un ion, un organite cellulaire, un lipide, et un polyoside.

11. Procédé selon l'une quelconque des revendications précédentes, **caractérisé par le fait que** le marqueur du potentiel sensibilisant et/ou irritant et/ou allergène est choisi parmi les molécules du complexe majeur d'histocompatibilité (MHC) de classe II ou les cytokines comme l'interleukine-1α, l'interleukine-1β, le facteur de stimulation de colonies (Granulocyte/Macrophage- Colony Stimulating Factor ou GM-CSF), le facteur de nécrose tumorale (Tumor Necrosis Factor ou TNF-α), la protéine interferon-induite 10 (interferon-induced protein 10 ou IP10) la protéine inflammatoire macrophagique de type 2 (macrophage inflammatory protein 2 ou MIP2) ou l'interferon-γ (IFN-γ).

12. Procédé selon l'une quelconque des revendications précédentes, **caractérisé par le fait que** le marqueur du potentiel sensibilisant et/ou irritant et/ou allergène est l'interleukine1-β.

13. Procédé selon l'une quelconque des revendications précédentes, **caractérisé par le fait que** le marqueur du potentiel sensibilisant et/ou irritant et/ou allergène est le taux d'expression des ARNm de l'interleukine1-β.

14. Procédé pour évaluer un produit capable de moduler voire inhiber l'effet sensibilisant et/ou irritant et/ou allergène d'un autre produit, **caractérisé par le fait que** le procédé selon l'une quelconque des revendications précédentes comprend une étape supplémentaire qui correspond à l'application d'un produit capable de moduler, voire inhiber, l'effet sensibilisant et/ou irritant et/ou allergène avant, pendant ou après la mise en contact du produit sensibilisant et/ou irritant et/ou allergène.

## Patentansprüche

1. Verfahren zur Bewertung des sensibilisierenden und/oder irritierenden und/oder allergenen Potenzials eines Produkts, **dadurch gekennzeichnet, dass** zumindest Keratinocyten und Vorläufer von Langerhans-Zellen gemeinsam während einer Zeitspanne kultiviert werden, die ausreicht, um die Differenzierung der Vorläufer der Langerhans-Zellen auszulösen, die Kultur und das zu testende Produkt während einer hinreichend langen Zeit miteinander in Kontakt gebracht werden, die Veränderung eines Markers für das sensibilisierende und/oder irritierende und/oder allergene Potenzial des Produkts bestimmt wird und die Ergebnisse dieser Bestimmung im Verhältnis zu einer Kontrollprobe bewertet werden.

2. Verfahren nach dem vorhergehenden Anspruch, **dadurch gekennzeichnet, dass** das Verhältnis der Anzahl von Keratinocyten zu den Vorläufern von Langerhans-Zellen im Bereich von 95/5 bis 25/75 in Prozent der Gesamtzahl der in der Co-Kultur befindlichen Zellen und vorzugsweise im Bereich von 75/25 bis 35/65 liegt und dass dieses Verhältnis noch bevorzugter 50/50 ist.

3. Verfahren nach einem der vorhergehenden Ansprüche, **dadurch gekennzeichnet, dass** die Keratinocyten durch Kultivierung aus dissoziierter Epidermis, die von einer Probe von normaler oder krankhafter Haut stammt, oder durch Kultivierung von Keratinocyten, die aus der Wurzelscheide von normalen oder krankhaften Haarfollikeln stammen, gewonnen werden.

4. Verfahren nach dem vorhergehenden Anspruch, **dadurch gekennzeichnet, dass** die Keratinocyten aus einer Probe von normaler oder krankhafter Haut gewonnen werden.

5. Verfahren nach einem- der vorhergehenden Ansprüche, **dadurch gekennzeichnet, dass** es sich bei den Keratinocyten um menschliche Keratinocyten handelt.

6. Verfahren nach einem der vorhergehenden Ansprüche, **dadurch gekennzeichnet, dass** die Vorläufer der Langerhans-Zellen ausgehend von Knochenmark, peripherem Blut oder Nabelschnurblut gereinigt werden.

7. Verfahren nach dem vorhergehenden Anspruch, **dadurch gekennzeichnet, dass** die Vorläufer der Langerhans-Zellen ausgehend von Nabelschnurblut gereinigt werden.

8. Verfahren nach einem der vorhergehenden Ansprüche, **dadurch gekennzeichnet, dass** es sich bei den Vorläufern von Langerhans-Zellen um hämatopoetische CD34+ Zellen handelt.

9. Verfahren nach einem der vorhergehenden Ansprüche, **dadurch gekennzeichnet, dass** die Vorläufer von Langerhans-Zellen menschlichen Ursprungs sind.

10. Verfahren nach einem der vorhergehenden Ansprüche, **dadurch gekennzeichnet, dass** der Marker für das sensibilisierende und/oder irritierende und/oder allergene Potenzial unter Nucleinsäuren, Proteinen oder Gruppen von Proteinen, die gegebenenfalls gebunden sind, Ionen, Zellorganellen, Lipiden und Polysacchariden ausgewählt ist.

11. Verfahren nach einem der vorhergehenden Ansprüche, **dadurch gekennzeichnet, dass** der Marker für das sensibilisierende und/oder irritierende und/oder allergene Potenzial unter den Molekülen des Haupt-Histokompatibilitäts-Komplexes (MHC) der Klasse II oder den Cytokinen, wie Interleukin-1α, Interleukin-1β, dem Kolonie-stimulierenden Faktor (Granulocyte/Macrophage- Colony Stimulating Factor oder GM-CSF), dem Tumor-Nekrose-Faktor (Tumor Necrosis Factor oder TNF-α), dem Interferon-induzierten Protein 10 (interferon-induced protein 10 oder IP10), dem inflammatorischen Makrophagen-Protein vom Typ 2 (macrophage inflammatory protein 2 oder MIP2) oder Interferon-γ (IFN-γ), ausgewählt ist.

12. Verfahren nach einem der vorhergehenden Ansprüche, **dadurch gekennzeichnet, dass** der Marker für das sensibilisierende und/oder irritierende und/oder allergene Potenzial Interleukin-1β ist.

13. Verfahren nach einem der vorhergehenden Ansprüche, **dadurch gekennzeichnet, dass** der Marker für das sensibilisierende und/oder irritierende und/oder allergene Potenzial der Grad der Expression der mRNAs von Interleukin-1β ist.

14. Verfahren zur Bewertung eines Produkts, das dazu befähigt ist, die sensibilisierende und/oder irritierende und/oder allergene Wirkung eines anderen Produkts zu modulieren oder sogar zu inhibieren, **dadurch gekennzeichnet, dass** das Verfahren nach einem der vorhergehenden Ansprüche einen zusätzlichen Schritt umfasst, bei dem ein Produkt, das dazu befähigt ist, die sensibilisierende und/oder irritierende und/oder allergene Wirkung zu modulieren oder sogar zu inhibieren, appliziert wird, bevor, während oder nachdem der Kontakt mit dem sensibilisierenden und/oder irritierenden und/oder allergenen Produkt erfolgt.

## Claims

1. Method for evaluating the sensitizing and/or irritant and/or allergenic potential of a product, **characterized in that** at least keratinocytes and Langerhans cell precursors are cultured simultaneously for a period of time sufficient to induce differentiation of the Langerhans cell precursors, **in that** the culture and the test product are brought into contact for a sufficient period of time, **in that** the variation of a marker for the sensitizing and/or irritant and/or allergenic potential of said product is measured, and **in that** the results of this measuring are evaluated compared with a control.

2. Method according to the preceding claim, **characterized in that** the ratio of the number of keratinocytes to the number of Langerhans cell precursors is between the ratios 95/5 and 25/75 as a percentage of the total number of cells in the coculture, and preferentially between 75/25 and 35/65, and even more preferentially this ratio is 50/50.

3. Method according to any one of the preceding claims, **characterized in that** the keratinocytes are obtained by culturing using dissociated epidermis originating from a normal or pathological skin sample, or by culturing keratinocytes derived from the sheath of normal or pathological hair follicles.

4. Method according to the preceding claim, **characterized in that** the keratinocytes are obtained from a normal or pathological skin sample.

5. Method according to any one of the preceding claims, **characterized in that** the keratinocytes are human keratinocytes.

6. Method according to any one of the preceding claims, **characterized in that** the Langerhans cell precursors are purified from bone marrow, from peripheral blood or from umbilical cord blood.

7. Method according to the preceding claim, **characterized in that** the Langerhans cell precursors are purified from umbilical cord blood.

8. Method according to any one of the preceding claims, **characterized in that** the Langerhans cell precursors are CD34⁺ haematopoietic cells.

9. Method according to any one of the preceding claims, **characterized in that** the Langerhans cell precursors are of human origin.

10. Method according to any one of the preceding claims, **characterized in that** the marker for the sensitizing and/or irritant and/or allergenic potential is chosen from a nucleic acid, a protein or a group of proteins which may or may not be linked, an ion, a cellular organelle, a lipid and a polysaccharide.

11. Method according to any one of the preceding claims, **characterized in that** the marker for the sensitizing and/or irritant and/or allergenic potential is chosen from major histocompatibility complex (MHC) class II molecules or cytokines, such as interleukin-1α, interleukin-1β, granulocyte/macrophage-colony stimulating factor (GM-CSF), tumour necrosis factor (TNF-α), interferon-induced protein 10 (IP10), macrophage inflammatory protein 2 (MIP2) or γ-interferon (γ-IFN).

12. Method according to any one of the preceding claims, **characterized in that** the marker for the sensitizing and/or irritant and/or allergenic potential is interleukin-1β.

13. Method according to any one of the preceding claims, **characterized in that** the marker for the sensitizing and/or irritant and/or allergenic potential is the level of expression of interleukin-1β mRNAs.

14. Method for evaluating a product capable of modulating, or even inhibiting, the sensitizing and/or irritant and/or allergenic effect of another product, **characterized in that** the method according to any one of the preceding claims comprises an additional step which corresponds to the application of a product capable of modulating, or even inhibiting, the sensitizing and/or irritant and/or allergenic effect before, during or after the sensitizing and/or irritant and/or allergenic product is brought into contact.
